# FASCICULE DE BREVET EUROPEEN

(11) **EP 1 360 677 B1**
(45) Date de publication et mention de la délivrance du brevet: **26.09.2007**
(21) Numéro de dépôt: 02700345.8
(22) Date de dépôt: 21.01.2002
(51) Int. Cl.: G09B 23/30, A01N 1/00

(54) **DISPOSITIF PERMETTANT DE CARACTERISER LA DIFFUSION D'UN PRODUIT SOUS FORME DISCRETE DANS UNE OU PLUSIEURS CAVITES CORPORELLES**
VORRICHTUNG ZUR BESTIMMUNG DER DIFFUSION EINES PRODUKTES INNERHALB EINER KÖRPERHÖHLE
DEVICE FOR CHARACTERISING DISCRETE DIFFUSION OF A PRODUCT IN ONE OR SEVERAL CORPOREAL CAVITIES

(30) Priorité: 24.01.2001 FR 0100952
(43) Date de publication de la demande: 12.11.2003
(73) Titulaire: Becton Dickinson France, 38800 Le Pont de Claix (FR)
(72) Inventeur: DURAND, Marc, F-43770 CHADRAC (FR); VEDRINE, Lionel, F-38400 SAINT-MARTIN D'HERES (FR); LAURENT, Philippe, F-69600 OULLINS (FR)
(74) Mandataire: Brédeville, Odile Marie
(86) Numéro de dépôt international: PCT/FR2002/000232
(87) Numéro de publication internationale: WO 2002/058462

(56) Documents cités:
- JP-A- 63 208 501
- US-A- 4 320 157
- DURAND M ET AL: "Preliminary study of the deposition of aerosol in the maxillary sinuses using a plastinated model." JOURNAL OF AEROSOL MEDICINE, vol. 14, no. 1, avril 2001 (2001-04), pages 83-93, XP001119132 ISSN: 0894-2684
- BACHERT C ET AL: "Experimental studies on the relationship between maxillary sinus ventilation and various obstructions of the nose and nasopharynx." RHINOLOGY, vol. 27, no. 1, mars 1989 (1989-03), pages 37-43, XP001119136 ISSN 0300-0729
- LENNON S ET AL: "Experiments on particle deposition in the human upper respiratory system" AEROSOL SCIENCE AND TECHNOLOGY, vol. 28, no. 5, 1998, pages 464-474, XP002222310 ISSN 0278-6826
- CORCORAN T E ET AL: "Characterization of the laryngeal jet using phase Doppler interferometry." JOURNAL OF AEROSOL MEDICINE, vol. 13, no. 2, juillet 2000 (2000-07), pages 125-137, XP009001146 ISSN 0894-2684
- UNNO T ET AL: "Distribution of beclomethasone dipropionate aerosol particles in the nasal cavity" JOURNAL OF OTOLARYNGOLOGY OF JAPAN, vol. 85, no. 3, 1982, pages 277-282, XP009001145 TOKYO, JAPAN

## Description

La présente invention concerne un dispositif permettant de caractériser la diffusion et/ou distribution d'au moins un produit ou substance d'intérêt sous forme discrète, liquide ou solide, dans une ou plusieurs cavités corporelles, notamment une cavité nasale et/ou un sinus.

Les produits sous forme discrète, notamment les aérosols, sont utilisés de manière courante pour traiter les affections de la sphère ORL.

Des études ont été réalisées par le passé pour déterminer quelles sont les zones d'une cavité nasale ou d'un sinus recevant un produit sous forme d'aérosol injecté depuis une narine. Ces études, conduites à partir de modèles anatomiques synthétiques reproduisant, autant que faire se peut, l'anatomie d'une cavité nasale ou d'un sinus, n'ont toutefois permis d'obtenir que des résultats relativement peu précis et peu fiables, non susceptibles d'être exploités de manière systématique.

Il a également été envisagé d'utiliser, sur des sujets ou patients, in vivo, des techniques de repérage optique de la diffusion d'un produit, soit par scintigraphie, soit par vision directe. Ces techniques présentent l'une comme l'autre des limitations notables quant à la possibilité d'exploiter les résultats obtenus.

La présente invention vise à remédier à ces inconvénients essentiels, en fournissant un dispositif à même de permettre une étude précise et fiable des zones ou sites d'une cavité corporelle susceptibles d'être effectivement atteints par un produit d'intérêt, notamment sous forme discrète, ce produit étant introduit, par exemple inhalé, à partir d'un orifice relié à cette cavité, selon des paramètres d'introduction déterminés ; ces paramètres d'introduction se rapportent notamment à la forme et aux dimensions du dispositif d'introduction, à la forme du jet de produit, à la position et l'orientation de ce dispositif d'introduction par rapport à l'orifice naturel communiquant directement ou indirectement avec la cavité étudiée, aux dimensions des particules du produit liquide ou solide, à la pression d'introduction, ou encore aux caractéristiques physico-chimiques du produit.

L'invention a en particulier pour objectif d'évaluer la diffusion du produit dans la sphère ORL d'un sujet, lorsque ce produit est injecté dans les cavités nasales à partir d'une narine ou dans la gorge à partir de la bouche.

L'invention a également pour objectif d'évaluer la possibilité d'administrer diverses substances sous forme discrète, dans un corps humain ou animal, par injection dans une cavité corporelle, à des fins de traitement thérapeutique ou de vaccination par exemple, et en particulier d'administrer des substances à action systémique, non directement liée au traitement d'affections de la sphère ORL, par introduction et diffusion dans les cavités nasales.

Le dispositif selon l'invention peut également être mis en oeuvre pour évaluer les conséquences de l'inhalation de produits polluants ou toxiques, sous forme discrète.

Selon l'invention, le dispositif comprend :
- une portion anatomique d'une partie observée d'un sujet ex vivo, comprenant au moins en partie la ou les cavités corporelles, et comprenant un orifice naturel avec lequel communique au moins une desdites cavités corporelles ; cette portion anatomique est obtenue par au moins une coupe de ladite partie observée, passant par la ou les cavités corporelles, et est traitée pour sa conservation, par exemple par une technique dite "de plastination", qui consiste à extraire l'eau et la graisse des tissus de cette portion anatomique et à les remplacer par au moins une matière synthétique imputrescible, par exemple une résine thermodurcie, rigide ou souple ;
- au moins un capteur apte à détecter et/ou quantifier ledit produit au niveau d'au moins un site ou zone de la ou des cavités corporelles ;
- au moins une pièce de fermeture placée contre ladite portion anatomique au niveau de chaque surface de coupe, de manière à fermer chaque cavité corporelle ou partie de cavité corporelle mais à laisser un accès audit orifice naturel, et
- éventuellement un dispositif d'introduction placé en regard dudit orifice naturel, ou engagé partiellement dans cet orifice, apte à réaliser une introduction dudit produit selon des paramètres prédéterminés.

Par "introduction", on entend aussi bien une injection, une projection, qu'une pulvérisation, ou tout autre mode de diffusion du produit d'intérêt, directement ou indirectement, au sein de la cavité corporelle étudiée.

La conformation de certaines cavités corporelles, en particulier des cavités nasales et des sinus, est dans certains cas très complexe sur le plan anatomique, et ne peut en aucune manière être reproduite fidèlement par un modèle synthétique. Au contraire, la technique de plastination appliquée à un sujet ex vivo permet de conserver dans une très large mesure la conformation anatomique de ces cavités, en particulier des cavités nasales et des sinus ; de plus, cette technique permet de conserver les muqueuses, qui apparaissent avoir une incidence notable sur la diffusion d'un produit sous forme discrète dans une cavité corporelle.

L'emploi d'un modèle plastiné du nez humain a déjà été proposé pour l'étude de la ventilation du sinus dans le document RHINOLOGY, vol. 27, no. 1, mars 1989, pages 37-43, XP001119136, ISSN 0300-0729. Pour caractériser la diffusion des particules fluorescentes dans une cavité nasale et/ou sinus, les auteurs du document AEROSOL SCIENCE AND TECHNOLOGY, vol. 28, no. 5, 1998, pages 464-474, XP002222310, ISSN 0278-6826 ont eu recours à un modèle anatomique synthétique.

La technique de plastination utilisée dans le cadre de l'invention consiste à opérer une fixation des tissus dans une solution de formol, puis une déshydratation dans de l'acétone à basse température, pour remplacer l'eau et les graisses contenues dans les tissus, puis une imprégnation forcée par immersion dans un bain de matière synthétique imputrescible, par exemple une résine thermodurcie.

Le produit ou la substance d'intérêt peut notamment être sous forme de gouttelettes liquides, de particules solides ou semi-solides, de poudre, de vapeur, ou de microcapsules.

Au moins un capteur peut être placé dans une cavité corporelle ; il est alors avantageusement disposé de manière à affleurer avec la surface de la paroi dans laquelle il est placé, afin de ne pas risquer de perturber le flux de produit dans la ou les cavités à étudier.

Ce capteur peut être de tout type approprié pour détecter la présence du produit d'intérêt. Il peut notamment s'agir d'un capteur d'humidité, d'un capteur optique, ou d'un capteur à impact.

Le dispositif peut également comprendre au moins un capteur situé non pas dans une cavité corporelle, mais à l'extérieur de celle-ci, par exemple dans la portion anatomique étudiée, ce capteur pouvant être mobile. Il peut notamment s'agir d'une caméra ou d'un endoscope.

La ou les pièces de fermeture précitées sont avantageusement amovibles, par exemple pour permettre de nettoyer ou de sécher la ou les cavités corporelles entre deux injections de produit.

Cette ou ces pièces peuvent être avantageusement transparentes, afin de permettre de visualiser la diffusion du produit pendant et après son introduction, et de détecter des fuites éventuelles.

Au moins une de ces pièces peut être opaque à un rayonnement du type α, β, γ, UV ou IR, par exemple à des fins de protection de l'utilisateur du dispositif.

De préférence, le dispositif comprend un dispositif d'introduction et des moyens réglables de positionnement dudit dispositif, permettant le réglage de la position et/ou de l'orientation de ce dispositif par rapport à ladite portion anatomique.

Selon une application préférentielle de l'invention, ladite portion anatomique est une portion de tête humaine. La ou lesdites cavités corporelles sont alors préférentiellement une ou plusieurs cavités nasales et/ou sinus. Le dispositif selon invention est ainsi particulièrement destiné à permettre de caractériser la diffusion d'un produit sous forme discrète, notamment un aérosol, dans une cavité nasale et/ou un sinus d'un sujet, lorsque ce produit est injecté à partir d'une narine.

La tête peut être coupée selon un plan antéro-postérieur médian, passant immédiatement au-delà de la cloison nasale. Cette cloison nasale est ainsi éliminée pour ne pas gêner la mise en place des capteurs.

La tête peut également être coupée selon un plan antéro-postérieur passant au niveau du sinus maxillaire, par exemple sensiblement dans le plan passant par la pupille.

La tête peut être coupée, pour obtenir la portion anatomique étudiée, selon tout ou tous plans de coupe appropriés pour l'étude de la cavité observée, par exemple système auditif, sinus, etc.

De préférence, la tête est coupée selon ces deux plans, par exemple respectivement antéro-postérieur médian et antéro-postérieur, pour permettre de caractériser la diffusion du produit d'intérêt simultanément dans une cavité nasale et dans le sinus maxillaire.

Le réglage du dispositif d'introduction permet l'obtention de résultats d'introduction et/ou diffusion, dans diverses positions ou orientations dudit dispositif par rapport à la portion anatomique, ou dans toute portion ou orientation par rapport à tout référentiel bidimensionnel ou tridimensionnel.

Avantageusement, dans ce cas, les moyens réglables de positionnement sont équipés d'au moins une graduation et d'au moins un repère permettant de régler la position et/ou l'orientation du dispositif d'introduction.

Le dispositif selon l'invention peut également comprendre une unité de traitement des signaux émis par le ou les capteurs, apte à délivrer un ou des signaux représentatifs de la présence et/ou concentration en produit sous forme discrète au niveau du ou desdits capteurs.

Le dispositif selon l'invention peut comprendre en outre des moyens propres à générer un mouvement de fluide (liquide ou gazeux), dans la ou lesdites cavités corporelles.

Ces moyens permettent de recréer un flux respiratoire ou inhalatoire. Il peut notamment s'agir d'un respirateur artificiel d'anesthésie.

Pour sa bonne compréhension, l'invention est à nouveau décrite ci-dessous en référence au dessin schématique annexé représentant, à titre d'exemple non limitatif, une forme de réalisation préférée du dispositif qu'elle concerne.
La figure 1 est une vue d'une portion de tête humaine traitée par "plastination" et coupée notamment selon un plan antéro-postérieur sensiblement médian ; et
la figure 2 est une vue en perspective du dispositif selon l'invention.

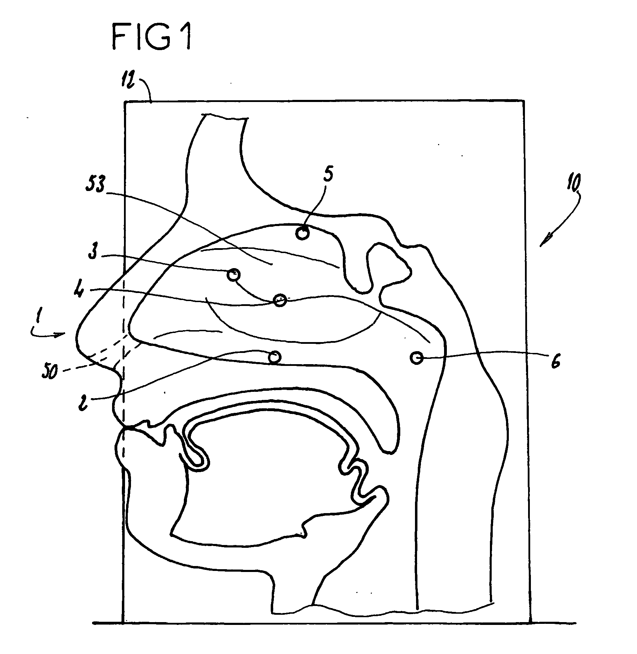

La figure 1 montre une section de tête humaine 1 coupée selon, d'une part, un plan antéro-postérieur médian 51 et, d'autre part, un plan 52 passant sensiblement par la zone médiane du sinus maxillaire. La coupe médiane a été réalisée immédiatement au-delà de la cloison nasale, de manière à éliminer cette cloison mais à conserver le maximum de volume à la cavité nasale.

La section 1 a été obtenue à partir de la tête d'un sujet ex vivo ayant fait l'objet d'un traitement permettant sa conservation, par une technique dite "de plastination". Cette technique est par exemple la suivante :
1) Prélèvement d'une tête moins de quarante-huit heures après le décès du sujet ; en effet, la durée s'écoulant entre le moment du décès et le prélèvement influe directement sur la qualité de la pièce obtenue, la muqueuse nasale étant très fragile, surtout au niveau des cornets, qui possèdent des qualités vasoactives importantes.
2) Fixation de la tête dans une solution de formol à 10 %, pendant au moins quatre semaines et de préférence pendant trois mois, sous une température de l'ordre de 5°C.
3) Préparation anatomique (retrait du cerveau notamment) et réalisation des coupes précitées.
4) Immersion de la section 1 obtenue dans de l'acétone à - 25°C, pendant une durée suffisante pour que l'acétone viennent remplacer l'eau et les lipides dans les tissus, par substitution à froid. Cette durée est généralement de l'ordre de quatre semaines.
5) Immersion de la section 1 dans un bain de silicone fluide, sous une température de - 25° C et pendant une durée allant de vingt-quatre à quarante-huit heures. Le silicone est par exemple celui dénommé "S10" commercialisé sous la marque "BIODUR".
6) Application progressive, alors que la section 1 est toujours immergée, d'une dépression atmosphérique pendant dix à vingt jours, de manière à extraire l'acétone des tissus et induire l'entrée du silicone dans ces tissus.
7) Egouttage du silicone pendant quarante-huit heures et polymérisation de ce silicone.

Le dichlorométhane peut être utilisé en lieu et place de l'acétone.

Des résines époxy peuvent être utilisées en lieu et place du silicone.

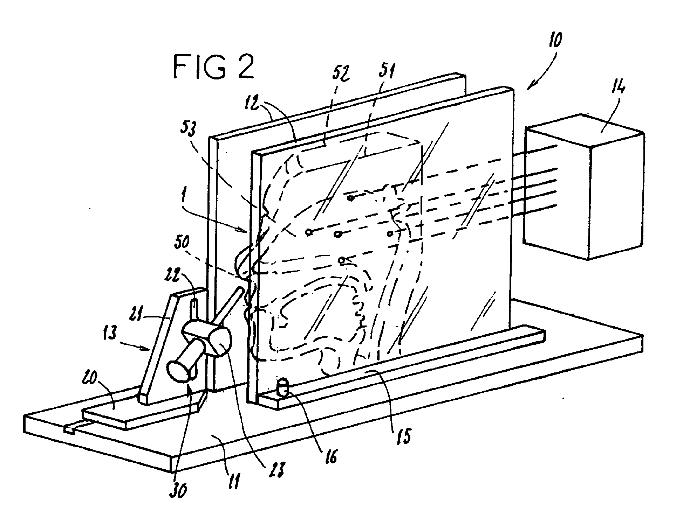

Dans l'exemple représenté, cinq capteurs d'humidité de type capacitif ont été mis en place sur la pièce 1, à savoir (cf. figure 2) :
- un capteur 2 au niveau de la lame horizontale du palatin,
- un capteur 3 au niveau du cornet moyen,
- un capteur 4 au niveau du méat moyen,
- un capteur 5 au niveau de la lame criblée, et
- un capteur 6 au niveau du cavum.

Ces capteurs 2 à 6 peuvent notamment être ceux disponibles sous la dénomination "LJK technologie ®".

La figure 2 montre un dispositif 10 permettant, à l'aide de la pièce 1 équipée des capteurs 2 à 6, de caractériser la diffusion d'un produit sous forme discrète, notamment un aérosol, dans la cavité nasale et/ou le sinus maxillaire de la section 1.

Ainsi que cela est représenté, le dispositif 10 comprend une plaque inférieure 11, ou embase, deux plaques 12 parallèles et perpendiculaires à la plaque 11, recevant entre elles la section 1 ou portion de tête, équipée des capteurs 2 à 6, des moyens 13 de support et de positionnement d'un dispositif d'introduction 30, et une unité 14 de traitement des signaux transmis par les capteurs 2 à 6.

La plaque 11 comprend des trous taraudés pour le montage de réglettes 15 de positionnement des plaques 12 au moyen de vis 16.

Les plaques 12 sont par exemple en matériau transparent de type "plexiglas". Elles sont appliquées étroitement sur les faces de la section 1 qui résultent des coupes précitées, et permettent de refermer la cavité nasale 53 et le sinus maxillaire de cette pièce 1. Un joint peut être mis en place entre la section 1 et les plaques 12.

Les moyens 13 comprennent une embase 20 fixée par des vis à la plaque 11, une aile 21 dans laquelle est aménagée une lumière 22, et un support 23, ce dernier comportant le dispositif d'introduction 30.

Le support 23 prend appui contre l'aile 21 et reçoit, dans un trou taraudé qu'il comprend, une vis engagée à coulissement au travers de la lumière 22, cette vis prenant appui par sa tête contre l'aile 21 du côté opposé au support 23. Le desserrage de cette vis permet de régler la hauteur et l'inclinaison du dispositif d'introduction 30 dans un plan vertical.

L'aile 21 et/ou le support 23 peuvent être équipés d'une graduation et de repères permettant de régler par exemple ladite hauteur et/ou ladite inclinaison sur la base de valeurs précises.

Les capteurs 2 à 6 sont reliés à l'unité de traitement 14. Celle-ci traite les signaux émis par ces capteurs, et délivre à une unité électronique (non représentée) des signaux représentatifs de la présence et/ou concentration, au niveau desdits capteurs 2 à 6, en produit d'intérêt introduit et diffusé.

Ainsi qu'il apparaît de ce qui précède, invention fournit un dispositif permettant de caractériser de manière fiable et précise la diffusion d'un produit sous forme discrète dans une ou plusieurs cavités corporelles, notamment une cavité nasale et/ou un sinus.

Il va de soi que l'invention n'est pas limitée à la forme de réalisation décrite ci-dessus à titre d'exemple mais qu'elle en embrasse au contraire toutes les variantes de réalisation entrant dans le champ de protection défini par les revendication ci-annexées.

D'autres cavités corporelles, telles que poumons, gorge, estomac, peuvent être étudiées et caractérisées avec tout dispositif construit ou obtenu selon la présente invention.

## Revendications

1. - Dispositif permettant de caractériser la diffusion d'un produit d'intérêt sous forme discrète dans une ou plusieurs cavités corporelles (53) au moins, notamment une cavité nasale et/ou un sinus, **caractérisé en ce qu'**il comprend :
- une portion anatomique (1) d'une partie observée d'un sujet ex vivo, comprenant au moins en partie la ou les cavités corporelles (53), et comprenant un orifice naturel (50) avec lequel communique au moins une desdites cavités corporelles ; cette portion anatomique (1) est obtenue par au moins une coupe (52, 51) de ladite partie observée, passant par la ou les cavités corporelles, et est traitée pour sa conservation, par exemple par une technique dite "de plastination", qui consiste à extraire l'eau et la graisse des tissus de cette portion anatomique (1) et à les remplacer par au moins une matière synthétique imputrescible, par exemple une résine thermodurcie ;
- au moins un capteur (2 à 6) apte à détecter et/ou quantifier ledit produit au niveau d'au moins un site ou zone de la ou des cavités corporelles ;
- au moins une pièce de fermeture (12) placée contre ladite portion anatomique (1) au niveau de chaque surface de coupe, de manière à fermer chaque cavité corporelle (53) ou partie de cavité corporelle, mais à laisser un accès audit orifice naturel (50), et
- éventuellement un dispositif d'introduction (30) placé en regard dudit orifice naturel, ou engagé partiellement dans cet orifice, apte à réaliser une introduction dudit produit selon des paramètres prédéterminés.

2. - Dispositif selon la revendication 1, **caractérisé en ce qu'**au moins un capteur (2 à 6) est placé dans une cavité corporelle (53) et est disposé de manière à affleurer avec la surface de la paroi dans laquelle il est placé.

3. - Dispositif selon la revendication 2, **caractérisé en ce qu'**il comprend au moins un capteur (2 à 6) situé à l'extérieur d'une cavité corporelle, par exemple dans la portion anatomique, ce capteur pouvant être mobile.

4. - Dispositif selon la revendication 1, **caractérisé en ce que** la ou les pièces de fermeture (12) sont amovibles.

5. - Dispositif selon l'une des revendications 1 à 4, **caractérisé en ce que** la ou les pièces de fermeture (12) sont transparentes.

6. - Dispositif selon l'une des revendications 1 à 5, **caractérisé en ce que** ladite portion anatomique (1) est une portion de tête humaine.

7. - Dispositif selon la revendication 6, **caractérisé en ce que** la ou lesdites cavités corporelles (53) sont une ou plusieurs cavités nasales et/ou sinus.

8. - Dispositif selon la revendication 7, **caractérisé en ce que** ladite tête (1) est coupée selon un plan antéro-postérieur médian (51), passant immédiatement au-delà de la cloison nasale.

9. - Dispositif selon la revendication 7 ou la revendication 8, **caractérisé en ce que** ladite tête (1) est coupée selon un plan antéro-postérieur (52) passant au niveau du sinus maxillaire, par exemple sensiblement dans le plan passant par la pupille.

10. - Dispositif selon l'une des revendications 1 à 9, **caractérisé en ce qu'**il comprend un dispositif d'introduction (30) du produit d'intérêt et des moyens (13) réglables de positionnement dudit dispositif, permettant le réglage de la position et/ou de l'orientation de ce dispositif par rapport à ladite portion anatomique (1).

11. - Dispositif selon la revendication 10, **caractérisé en ce que** les moyens réglables (13) de positionnement sont équipés d'au moins une graduation et d'au moins un repère permettant de régler la position et/ou l'orientation du dispositif d'introduction (30).

12. - Dispositif selon l'une des revendications 1 à 11, **caractérisé en ce qu'**il comprend une unité (14) de traitement des signaux émis par le ou les capteurs (2 à 6), apte à délivrer un ou des signaux représentatifs de la présence et/ou concentration en produit au niveau du ou desdits capteurs (2 à 6).

13. - Dispositif selon l'une des revendications 1 à 12, **caractérisé en ce qu'**il comprend des moyens propres à générer un mouvement de fluide (liquide ou gazeux) dans la ou lesdites cavités corporelles.

14. - Dispositif selon la revendication 12 ou la revendication 13, **caractérisé en ce qu'**il comprend une plaque inférieure (11), deux plaques (12) recevant entre elles la portion anatomique (1) équipée de capteurs (2 à 6), lesdits moyens réglables (13) de positionnement du dispositif d'introduction (30), et ladite unité (14) de traitement des signaux transmis par les capteurs (2 à 6).

## Claims

1. A device for characterizing the diffusion of a product of interest in discrete form in one or more body cavities (53) at least, in particular a nasal cavity and/or a sinus, wherein said device comprises:
- an anatomical portion (1) of an observed part of a dead subject, comprising at least in part the body cavity or cavities (53), and comprising a natural orifice (50) with which at least one of said body cavities communicates; this anatomical portion (1) is obtained by at least one section (52, 51) through said observed part, passing through the body cavity or cavities, and is treated for its preservation, for example by a technique called "plastination", which consists in extracting water and fat from the tissues of this anatomical portion (1) and replacing them with at least one nonputrefying synthetic material, for example a thermoset resin;
- at least one sensor (2 to 6) which can detect and/or quantify said product at at least one site or zone of the body cavity or cavities;
- at least one closure piece (12) placed against said anatomical portion (1) in the area of each sectioned surface, in such a way as to close each body cavity (53) or part of the body cavity, but to leave access to said natural orifice (50), and
- if appropriate, an introduction device (30) placed opposite said natural orifice, or engaged partially in this orifice, and able to introduce said product in accordance with predetermined parameters.

2. The device as claimed in claim 1, wherein at least one sensor (2 to 6) is placed in a body cavity (53) and is arranged so as to be flush with the surface of the wall in which it is placed.

3. The device as claimed in claim 2, wherein said device comprises at least one sensor (2 to 6) situated outside a body cavity, for example in the anatomical portion, this sensor being movable.

4. The device as claimed in claim 1, wherein the closure piece or pieces (12) are removable.

5. The device as claimed in one of claims 1 through 4, wherein the closure piece or pieces (12) are transparent.

6. The device as claimed in one of claims 1 through 5, wherein said anatomical portion (1) is a portion of the human head.

7. The device as claimed in claim 6, wherein said body cavity or cavities (53) are one or more nasal cavities and/or sinuses.

8. The device as claimed in claim 7, wherein said head (1) is sectioned on a median anteroposterior plane, (51) passing immediately behind the nasal septum.

9. The device as claimed in claim 7 or claim 8, wherein said head (1) is sectioned on an anteroposterior plane (52) passing through the area of the maxillary sinus, for example substantially in the plane passing through the pupil.

10. The device as claimed in one of claims 1 through 9, wherein said device comprises an introduction device (30) for the product of interest, and adjustable means (13) for positioning said device, making it possible to set the position and/or orientation of this device relative to said anatomical portion (1).

11. The device as claimed in claim 10, wherein the adjustable positioning means (13) are equipped with at least one graduation and at least one marker making it possible to set the position and/or orientation of the introduction device (30).

12. The device as claimed in one of claims 1 through 11, wherein said device comprises a unit (14) for processing the signals which are emitted by the sensor or sensors (2 to 6), able to deliver a signal or signals representing the presence and/or concentration of product in the area of said sensor or sensors (2 to 6).

13. The device as claimed in one of claims 1 through 12, wherein said device comprises means which are able to general a movement of fluid (liquid or gaseous) in said body cavity or cavities.

14. The device as claimed in claim 12 or claim 13, wherein said device comprises a lower plate (11), two plates (12) receiving between them the anatomical portion (1) equipped with sensors (2 to 6), said adjustable means (13) for positioning the introduction device (30), and said unit (14) for processing the signals transmitted by the sensors (2 to 6).

## Patentansprüche

1. Vorrichtung zum Charakterisieren der Diffusion eines interessierenden Produkts in diskreter Form zumindest in einer oder mehreren Körperhöhlen (53), insbesondere einer Nasenhöhle und/oder einem Sinus, **dadurch gekennzeichnet, dass** sie aufweist:
- einen anatomischen Abschnitt (1) eines betrachteten Teils einer Person ex vivo, der zumindest zum Teil die Körperhöhle oder Körperhöhlen (53) und eine natürliche Öffnung (50) aufweist, mit der zumindest eine der Körperhöhlen kommuniziert; wobei der anatomische Abschnitt (1) durch zumindest einen Schnitt (52, 51) des betrachteten Teils erhalten worden ist, der durch die Körperhöhle oder Körperhöhlen verläuft, und zu seiner Konservierung beispielsweise durch ein als "Plastination" bezeichnetes Verfahren behandelt worden ist, das darin besteht, Wasser und Fett der Gewebe dieses anatomischen Abschnitts (1) zu extrahieren und diese durch zumindest ein unverwesliches synthetisches Material zu ersetzen, beispielsweise durch einen warm ausgehärteten Harz;
- zumindest einen Messaufnehmer (2 bis 6), der in der Lage ist, das Produkt im Bereich zumindest einer Stelle oder Zone der Körperhöhle oder Körperhöhlen zu detektieren und/oder zu quantifizieren;
- zumindest ein Verschlussstück (12), das gegen den anatomischen Abschnitt (1) im Bereich jeder Schnittfläche angeordnet ist, derart, dass jede Körperhöhle (53) oder jedes Körperhöhlenteil geschlossen wird, wobei jedoch ein Zugang zu der natürlichen Öffnung (50) belassen wird, und
- gegebenenfalls eine Einführvorrichtung (30), die der natürlichen Öffnung zugewandt angeordnet ist oder partiell in diese Öffnung eingreift, und die in der Lage ist, eine Einführung des Produkts gemäß vorbestimmten Parametern zu ermöglichen.

2. Vorrichtung nach Anspruch 1, **dadurch gekennzeichnet, dass** zumindest ein Messaufnehmer (2 bis 6) in einer Körperhöhle (53) positioniert ist und derart angeordnet ist, dass er mit der Oberfläche der Wand, in der er positioniert ist, fluchtet.

3. Vorrichtung nach Anspruch 2, **dadurch gekennzeichnet, dass** sie zumindest einen Messaufnehmer (2 bis 6) aufweist, der sich außerhalb einer Körperhöhle befindet, beispielsweise in dem anatomischen Abschnitt, wobei dieser Messaufnehmer beweglich sein kann.

4. Vorrichtung nach Anspruch 1, **dadurch gekennzeichnet, dass** das oder die Verschlussstücke (12) abnehmbar ist/sind.

5. Vorrichtung nach einem der Ansprüche 1 bis 4, **dadurch gekennzeichnet, dass** das oder die Verschlussstücke (12) transparent ist/sind.

6. Vorrichtung nach einem der Ansprüche 1 bis 6, **dadurch gekennzeichnet, dass** der anatomische Abschnitt (1) ein Abschnitt eines menschlichen Kopfes ist.

7. Vorrichtung nach Anspruch 6, **dadurch gekennzeichnet, dass** die Körperhöhle oder Körperhöhlen (53) eine Nasenhöhle oder mehrere Nasenhöhlen und/ oder ein oder mehrere Sinus ist/ sind.

8. Vorrichtung nach Anspruch 7, **dadurch gekennzeichnet, dass** der Kopf (1) entlang einer antero-posterioren Mittelebene (51) geschnitten ist, die unmittelbar neben der Nasenscheidewand hindurchgeht.

9. Vorrichtung nach Anspruch 7 oder nach Anspruch 8, **dadurch gekennzeichnet, dass** der Kopf (1) entlang einer antero-posterioren Ebene (52) geschnitten ist, die im Bereich der Kieferhöhle (Sinus maxillaris) hindurchgeht, beispielsweise etwa in der Ebene, die durch die Pupille hindurchgeht.

10. Vorrichtung nach einem der Ansprüche 1 bis 9, **dadurch gekennzeichnet, dass** sie eine Einführvorrichtung (30) zum Einführen des interessierenden Produkts und einstellbare Mittel (13) zum Positionieren der Einführvorrichtung aufweist, die das Einstellen der Position und/oder der Orientierung dieser Einführvorrichtung bezüglich des anatomischen Abschnittes (1) ermöglichen.

11. Vorrichtung nach Anspruch 10, **dadurch gekennzeichnet, dass** die einstellbaren Mittel (13) zum Positionieren mit zumindest einer Skala und zumindest einer Markierung versehen sind, die es ermöglichen, die Position und/oder die Orientierung der Einführvorrichtung (30) einzustellen.

12. Vorrichtung nach einem der Ansprüche 1 bis 11, **dadurch gekennzeichnet, dass** sie eine Signalverarbeitungseinheit (14) zur Verarbeitung von Signalen aufweist, die von dem oder den Messaufnehmern (2 bis 6) geliefert werden, und die in der Lage ist, ein oder mehrere Signale auszugeben, die für das Vorhandensein und/oder die Konzentration des Produkts im Bereich des oder der Messaufnehmer (2 bis 6) repräsentativ sind.

13. Vorrichtung nach einem der Ansprüche 1 bis 12, **dadurch gekennzeichnet, dass** sie Mittel aufweist, die geeignet sind, eine Bewegung eines Fluids (flüssig oder gasförmig) in der Körperhöhle oder den Körperhöhlen zu erzeugen.

14. Vorrichtung nach Anspruch 12 oder nach Anspruch 13, **dadurch gekennzeichnet, dass** sie eine untere Platte (11), zwei Platten (12), die dazwischen den mit den Messaufnehmern (2 bis 6) versehenen anatomischen Abschnitt (1) aufnehmen, die einstellbaren Mittel (13) zum Positionieren der Einführvorrichtung (30) und die Signalverarbeitungseinheit (14) zur Verarbeitung der von den Messaufnehmern (2 bis 6) übermittelten Signale aufweist.
